# FASCICULE DE BREVET EUROPEEN

(11) **EP 0 935 960 B1**
(45) Date de publication et mention de la délivrance du brevet: **30.05.2001**
(21) Numéro de dépôt: 99400120.4
(22) Date de dépôt: 20.01.1999
(51) Int. Cl.: A61K 7/48, A61K 7/00

(54) **Utilisaton du 1,2-pentanediol dans une composition cosmétique ou dermatologique contenant une dispersion aqueuse de particules de polymére filmogène, et composition cosmétique ou dermatologique comprenant ces constituants**
Verwendung von 1,2-Pentandiol in einer kosmetischen oder dermatologischen Zusammensetzung, welche filmbildende Polymerpartikel in wässriger Dispersion enthält und kosmetische oder dermatologische Zusammensetzung, enthaltend obige Bestandteile
Use of 1,2-pentanediol in cosmetic or dermatologic composition containing an aqueous dispersion of polymeric film forming particles and cosmetic or dermatologic composition containing these components

(30) Priorité: 12.02.1998 FR 9801699
(43) Date de publication de la demande: 18.08.1999
(73) Titulaire: L'OREAL, 75008 Paris (FR)
(72) Inventeur: Agostini, Isabelle, 92290 Chatenay Malabry (FR); Cupferman, Sylvie, 75015 Paris (FR)
(74) Mandataire: Casalonga, Axel

(56) Documents cités:
- WO-A-96/11572
- WO-A-97/30692
- DE-A- 4 320 744

## Description

La présente invention concerne une composition cosmétique ou dermatologique susceptible d'être appliquée sur la peau, les semi-muqueuses, les muqueuses et/ou les fibres kératiniques. Cette composition comprend en particulier une dispersion aqueuse de particules de polymère filmogène et du 1,2-pentanediol, et peut être utilisée en tant que produit de soin et de maquillage.

Il est intéressant d'utiliser dans les compositions cosmétiques ou dermatologiques une dispersion aqueuse de particules de polymère filmogène comme le montrent par exemple les demandes de brevets japonais H8-225433 et H8-225434 et les demandes de brevets EP-A-0679384, EP-A-0687461 et EP-A-0775483.

De telles compositions contiennent des proportions importantes de phase aqueuse, généralement supérieures à 50%. Ces compositions aqueuses sont parfaitement tolérées par l'organisme. En contrepartie, la part importante qu'y occupe l'eau en fait des cibles privilégiées pour l'intrusion des moisissures, bactéries et autres germes. Cette contamination microbienne peut survenir au cours de la fabrication du produit et surtout au cours de son utilisation par la consommatrice et est particulièrement importante dans le cas des rouges à lèvres et des vernis à ongles aqueux qui nécessitent l'emploi d'un applicateur trempant en continu dans un flacon qui, du fait des fréquents va-et-vient dans le flacon contenant le produit, favorise la recontamination du produit à chaque utilisation.

Il s'est donc avéré nécessaire de mettre au point une protection antimicrobienne pour les compositions cosmétiques et dermatologiques contenant une dispersion aqueuse de particules de polymère filmogène.

La mise au point d'un système de protection antimicrobienne pour ce type de composition a été compliquée par de nombreuses contraintes quant au choix des agents antimicrobiens et notamment :
- des contraintes de législation, les agents antimicrobiens sélectionnés devant être autorisés pour une application sur les muqueuses et les semi-muqueuses;
- des contraintes de solubilité : en l'absence de phase grasse dans la formule, le système de protection antimicrobienne doit être totalement hydrosoluble;
- des contraintes de température de mise en oeuvre : l'hydrosolubilité de l'agent antimicrobien doit être totale à froid, la formule ne supportant pas le chauffage : lorsqu'on chauffe une dispersion aqueuse de particules de polymère, les particules floculent à une température supérieure à 45°C;
- des contraintes de pH : le système de protection antimicrobienne doit être efficace au pH de la formule, et notamment à des pH de 6 à 8,5, le pH d'une composition appliquée sur la peau étant généralement neutre et ce en l'absence de régulateur de pH acide; en effet, il est connu qu'à pH acide, la majorité des bactéries et champignons ne résiste pas;
- des contraintes de compatibilité avec la dispersion aqueuse de particules de polymère qui présente de nombreuses incompatibilités; par exemple, la chlorhexidine, qui est utilisée généralement comme conservateur en cosmétique et en dermatologie, ne peut être utilisée en présence d'une dispersion aqueuse de particules de polymère.

Après avoir effectué de nombreux essais pour parvenir à un système de protection antimicrobienne, notamment antibactérienne et/ou antifongique, satisfaisant à tous les critères ci-dessus, la demanderesse a découvert que le 1,2-pentanediol était parfaitement bien adapté pour être utilisé à titre d'agent antimicrobien dans une dispersion aqueuse de particules de polymère filmogène.

Le 1,2-pentanediol appelé aussi pentylèneglycol (dénomination CTFA) est connu en cosmétique comme bactéricide et fongicide (G. Proserpio et R. Cattaneo, Cosmetic and Toiletries, Ed. It. n ° 3/1996, 11-13, 16-19) et comme agent régulateur d'hydratation pour la peau (demande WO-A-95 01151). II est également décrit pour le traitement topique de la peau et du cuir chevelu en raison de son effet antimicrobien dans la demande WO-A-97 30692.

Néanmoins, il n'a jamais été utilisé en présence d'une dispersion aqueuse de particules de polymère.

La demanderesse a découvert que le 1,2-pentanediol, par ses propriétés antibactériennes et antifongiques, non seulement permet de protéger la dispersion aqueuse de particules de polymère, mais présente également des propriétés de plastifiant du polymère en dispersion, ce qui permet de limiter l'emploi des plastifiants classiques tels que d'autres glycols (glycérol, propylèneglycol) et donc de diminuer le toucher collant de la composition. Il présente aussi des propriétés d'hydratant, ce qui est important pour la peau et les lèvres, ainsi que des propriétés d'antigel et a en plus l'avantage de rendre la composition plus brillante que lorsqu'on utilise un conservateur classique, et ce, sans altérer les propriétés de longue tenue et/ou "sans transfert" de cette composition, ce qui est tout à fait surprenant et très recherché pour les vernis à ongles et les rouges à lèvres.

Ainsi, un objet de l'invention est l'utilisation dans une composition cosmétique ou dermatologique susceptible d'être appliquée sur la peau, les fibres kératiniques, les semi-muqueuses et/ou les muqueuses, d'une dispersion aqueuse de particules de polymère filmogène associée à un système de protection antimicrobienne, notamment antibactérienne et/ou antifongique, comprenant du 1,2-pentanediol.

Un autre objet de l'invention est l'utilisation pour maquiller, protéger et/ou traiter non thérapeutiquement et/ou pour la fabrication d'une composition destinée à traiter thérapeutiquement la peau, les fibres kératiniques, les semi-muqueuses et/ou les muqueuses, en particulier les lèvres du visage et le corps, d'une dispersion aqueuse de particules de polymère filmogène associée à un système de protection antimicrobienne, notamment antibactérienne et/ou antifongique, comprenant du 1,2-pentanediol.

Un autre objet de l'invention est une composition cosmétique ou dermatologique susceptible d'être appliquée sur la peau, les fibres kératiniques, les semi-muqueuses et/ou les muqueuses, comprenant une dispersion aqueuse de particules de polymère filmogène associée à un système de protection antimicrobienne, notamment antibactérienne et/ou antifongique, comprenant du 1,2-pentanediol.

Un autre objet de l'invention est une composition de maquillage des lèvres ou du corps comprenant une dispersion aqueuse de particules de polymère filmogène associée à un système de protection antimicrobienne, notamment antibactérienne et/ou antifongique, comprenant du 1,2-pentanediol.

Un autre objet de l'invention est un procédé de protection antimicrobienne d'une composition contenant une dispersion aqueuse de particules de polymère filmogène, consistant à introduire dans la composition un système de protection antimicrobienne, notamment antibactérienne et/ou antifongique, comprenant du 1,2-pentanediol.

On a constaté que la composition selon l'invention est facilement applicable et s'étale aisément et uniformément sur la peau, les semi-muqueuses et les muqueuses, en particulier sur les lèvres du visage et le corps.

La composition selon l'invention trouve une application particulièrement intéressante dans le domaine du soin et/ou du maquillage de la peau du visage et du corps, des fibres kératiniques, des muqueuses et/ou des semi-muqueuses. On entend notamment par muqueuse la partie interne de la paupière inférieure; par semi-muqueuses, on entend plus particulièrement les lèvres du visage; par fibres kératiniques, on entend notamment les cils, les sourcils, les cheveux et les ongles.

La composition selon l'invention permet l'obtention d'un film homogène, qui présente une texture légère et reste confortable à porter tout au long de la journée. Le film n'est pas du tout collant après séchage. II est "sans transfert" et/ou de longue tenue.

La composition selon l'invention trouve donc une application toute particulière en tant que composition à appliquer sur les lèvres et le corps, notamment en tant que rouge à lèvres et produit de maquillage semi-permanent du corps.

D'autre part, le film obtenu peut être très brillant, ou plus ou moins mat, selon la nature des constituants de la composition, d'où une gamme plus étendue de produits de maquillage, brillants ou mats, au choix.

La composition selon l'invention comprend au moins une dispersion aqueuse de particules de polymère filmogène. Elle peut se présenter sous forme de fluide aqueux ou hydroalcoolique, de pâte ou d'émulsion. Dans ce dernier cas, elle peut contenir des corps gras tels que des huiles et des cires.

Parmi les polymères filmogènes utilisables dans la présente invention, on peut citer les polymères synthétiques, de type polycondensats ou de type radicalaires, les polymères d'origine naturelle, et leurs mélanges.

On peut ainsi citer, parmi les polycondensats, les polyuréthannes anioniques, cationiques, non ioniques ou amphotères, les polyuréthannes acryliques, les polyuréthannes-polyvinylpyrrolidones, les polyester-polyuréthannes, les polyéther-polyuréthannes, les polyurées, les polyurée-polyuréthannes, et leurs mélanges. Le polyuréthanne peut être, par exemple, un copolymère polyuréthanne, polyurée/uréthanne ou polyurée, aliphatique, cycloaliphatique ou aromatique, comportant, seule ou en mélange,
- au moins une séquence d'origine polyester aliphatique linéaire ou ramifié et/ou cycloaliphatique et/ou aromatique, et/ou
- au moins une séquence d'origine polyéther aliphatique et/ou cycloaliphatique et/ou aromatique, et/ou
- au moins une séquence siliconée, substituée ou non, ramifiée ou non, par exemple polydiméthylsiloxane ou polyméthylphénylsiloxane, et/ou
- au moins une séquence comportant des groupes fluorés.

Les polyuréthannes tels que définis dans l'invention peuvent être également obtenus à partir de polyesters, ramifiés ou non, ou d'alkydes comportant des hydrogènes mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire.

On peut également citer les polyesters, les polyesters amides, les polyesters à chaîne grasse, les polyamides, et les résines époxyesters.

Les polyesters peuvent être obtenus, de façon connue, par polycondensation de diacides aliphatiques ou aromatiques avec des diols aliphatiques ou aromatiques ou des polyols. Comme diacides aliphatiques, on peut utiliser l'acide succinique, l'acide glutarique, l'acide adipique, l'acide pimélique, l'acide subérique ou l'acide sébacique. Comme diacides aromatiques, on peut utiliser l'acide téréphtalique ou l'acide isophtalique, ou bien encore un dérivé tel que l'anhydride phtalique. Comme diols aliphatiques, on peut utiliser l'éthylène glycol, le propylène glycol, le diéthylène glycol, le néopentyl glycol, le cyclohexane diméthanol, le 4,4'-(1-méthylpropylidène)bisphénol. Comme polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine. la méta- ou para-phénylènediamine. Comme aminoalcool, on peut utiliser la monoéthanolamine.

Comme monomère porteur de groupement anionique pouvant être utilisé lors de la polycondensation, on peut citer par exemple l'acide diméthylol propionique, l'acide trimellitique ou un dérivé tel que l'anhydride trimellitique, le sel de sodium de l'acide sulfo-3 pentanediol, le sel de sodium de l'acide 5-sulfo 1,3-benzène dicarboxylique.

Les polyesters à chaîne grasse peuvent être obtenus par l'utilisation de diols à chaîne grasse lors de la polycondensation.

Les résines époxyesters peuvent être obtenues par polycondensation d'acides gras avec un condensat aux extrémités α,ω-diépoxy.

Les polymères de type radicalaire peuvent être notamment des polymères, ou des copolymères, acryliques et/ou vinyliques. Ces polymères peuvent résulter de la polymérisation de monomères à insaturation éthylénique ayant au moins un groupement acide et/ou des esters de ces monomères acides et/ou des amides de ces monomères acides. On utilise de préférence des polymères radicalaires anioniques, c'est-à-dire préparés à partir d'au moins un monomère à groupement acide.

Comme monomère porteur de groupement acide pouvant être utilisé lors de la polymérisation radicalaire, on peut citer les acides carboxyliques insaturés α,β-éthyléniques tels que l'acide acrylique, l'acide méthacrylique, l'acide crotonique, l'acide maléique, l'acide itaconique ainsi que l'acide 2-acrylamido 2-méthyl propane sulfonique. On utilise de préférence l'acide (méth)acrylique.

Les polymères acryliques peuvent résulter de la copolymérisation de monomères choisis parmi l'acide acrylique ou méthacrylique et les esters et/ou les amides de l'acide acrylique ou de l'acide méthacrylique. Comme exemples de monomères de type ester, on peut citer plus particulièrement les méthacrylates d'alkyle, en particulier d'alkyle en C₁-C₂₀ et de préférence en C₁-C₈ tels que le méthacrylate de méthyle, le méthacrylate d'éthyle, le méthacrylate de butyle, le méthacrylate d'isobutyle, le méthacrylate de 2-éthyl hexyle, le méthacrylate de lauryle. Comme exemples de monomères de type amide, on peut citer le N-tert-butyl acrylamide et le N-tert-octyl acrylamide.

On peut utiliser des polymères acryliques obtenus par copolymérisation de monomères à insaturation éthylénique contenant des groupements hydrophiles, de préférence de nature non ionique, tels que l'acrylate d'hydroxyéthyle, l'acrylate de 2-hydroxypropyle, le méthacrylate d'hydroxyéthyle, le méthacrylate de 2-hydroxypropyle.

Les polymères vinyliques peuvent résulter de l'homopolymérisation ou de la copolymérisation de monomères choisis parmi les esters vinyliques, le styrène, l'a-méthylstyrène ou le butadiène. Comme exemples d'esters vinyliques, on peut citer l'acétate de vinyle, le néodécanoate de vinyle, le pivalate de vinyle, le benzoate de vinyle et le tert-butyl benzoate de vinyle. En particulier, ces monomères peuvent être polymérisés avec des monomères acides et/ou leurs esters et/ou leurs amides, tels que ceux mentionnés précédemment.

On peut également utiliser des copolymères acryliques/silicones, ou encore des copolymères nitrocellulose/acryliques.

La liste des monomères donnée n'est pas limitative et il est possible d'utiliser tout monomère connu de l'homme du métier dans les catégories de monomères acryliques et vinyliques.

Selon l'invention, on utilise de préférence comme polymère filmogène un copolymère choisi parmi les copolymères acide (méth)acrylique / (méth)acrylate, acide (méth)acrylique / α-méthyl styrène, (méth)acrylate / styrène, acide (méth)acrylique / styrène, (méth)acrylate / (méth)acrylate, (méth)acrylate / α-méthylstyrène. Préférentiellement, on utilise un copolymère issu de la copolymérisation de monomères méthacrylates d'alkyle en C₁-C₈, éventuellement associés à l'acide acrylique, au styrène et à l'α-méthylstyrène.

Les polymères d'origine naturelle, éventuellement modifiés, peuvent être choisis parmi la résine shellac, la gomme de sandaraque, les dammars, les élémis, les copals, les dérivés cellulosiques, et leurs mélanges.

On peut encore citer les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes. Ces polymères sont généralement appelés polymères hybrides.

La dispersion aqueuse comprenant un ou plusieurs polymères filmogènes peut être préparée par l'homme du métier sur la base de ses connaissances générales.

La teneur en matière sèche des dispersions aqueuses selon la présente invention peut être de l'ordre de 5-70% en poids, et de préférence 30-60%, par rapport au poids total de la dispersion.

La composition peut comprendre 1-60% en poids, de préférence 5-40% en poids de matière sèche de polymères filmogènes.

La taille des particules de polymères en dispersion aqueuse peut être comprise entre 5 et 500 nm, et est de préférence comprise entre 20 et 150 nm, ce qui permet d'obtenir un film ayant une brillance remarquable.

Le système de protection antimicrobienne selon l'invention comprend :
. notamment de 0,1 à 10% en poids, de préférence 1 à 7%, et de manière optimale de 3 à 5% de 1,2-pentanediol;
. 0 à 5% en poids, avantageusement 0,05 à 2%, et de préférence 0,2 à 1% de p-hydroxybenzoate de méthyle sodé; et
. 0 à 40% en poids, avantageusement 0,5 à 30%, et de préférence 1 à 10% d'alcool éthylique.

La composition peut, en outre, comprendre au moins un colorant hydrosoluble et/ou au moins un pigment, et/ou au moins une charge et/ou au moins un agent nacrant, utilisés de manière usuelle dans le domaine de la cosmétique et du maquillage.

Par pigments, il faut comprendre des particules blanches ou colorées, minérales ou organiques, insolubles dans le milieu, destinées à colorer et/ou opacifier la composition, ou à filtrer les rayons UV. Les pigments peuvent être présents dans la composition à raison de 0-35% en poids de la composition finale, et de préférence à raison de 1-20%. Ils peuvent être blancs ou colorés, minéraux et/ou organiques, de taille usuelle ou nanométrique. On peut citer, parmi les pigments et/ou nanopigments minéraux, les dioxydes de titane, de zirconium ou de cérium, ainsi que les oxydes de zinc, de fer ou de chrome et le bleu ferrique. Parmi les pigments organiques, on peut citer le noir de carbone, les laques de baryum, strontium, calcium (DC Red n°7), aluminium et le carmin de cochenille.

Parmi les colorants hydrosolubles, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, et leurs mélanges.

Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les nacres peuvent être présentes dans la composition à raison de 0 à 20% du poids total de la composition, de préférence à un taux élevé de l'ordre de 1 à 15%. Parmi les nacres utilisables dans l'invention, on peut citer le mica recouvert d'oxyde de titane, d'oxyde de fer, de pigment naturel ou d'oxychlorure de bismuth tel que le mica titane coloré.

Les charges peuvent être présentes à raison de 0 à 35% du poids total de la composition, de préférence 0,5 à 15%. On peut notamment citer le talc, le mica, le kaolin, les poudres de Nylon (Orgasol notamment) et de polyéthylène, le Téflon, l'amidon, le nitrure de bore, des microsphères de copolymères telles que l'Expancel (Nobel Industrie), le Polytrap (Dow Corning) et les microbilles de résine de silicone (Tospearl de Toshiba, par exemple).

On peut également ajouter dans la composition selon l'invention tout additif connu tel que des agents épaississants, par exemple des argiles, des gommes, des silices, des dérivés cellulosiques, un polymère synthétique tel qu'un polymère acrylique ou un polymère associatif de type polyuréthanne, une gomme naturelle telle que la gomme xanthane, des agents d'étalement, des dispersants, des agents anti-mousses, des filtres UV, des parfums, des actifs cosmétiques, pharmaceutiques ou dermatologiques, des vitamines et leurs dérivés, des matières biologiques et leurs dérivés, des tensio-actifs pour disperser les pigments, des cires, des huiles.

Bien entendu, l'homme du métier veillera à choisir ce ou ces éventuels additifs et/ou leur quantité, de manière telle que les propriétés avantageuses de la composition selon l'invention ne soient pas, ou substantiellement pas, altérées par l'adjonction envisagée.

Le pH de la composition finale obtenue est de préférence inférieur à 9,5, avantageusement compris entre 5 et 8,5, et de préférence entre 6 et 8.

Cette composition doit bien entendu être apte à se déposer sur un support tel que la peau, les semi-muqueuses ou les muqueuses et les fibres kératiniques.

La composition selon l'invention peut se présenter sous forme fluide, gélifiée, semi-solide, pâte souple, voire solide telle que de stick ou bâton.

Elle trouve en particulier une application en tant que produit de maquillage, notamment en tant que rouge à lèvres, fond de teint, fard à joues ou fard à paupières, eye-liner, mascara, vernis à ongles, ou encore produit de maquillage du corps du type tatouage provisoire ou semi-permanent. On peut également envisager une application dans le domaine des compositions de soin, des compositions solaires ou autobronzantes, des compositions dermatologiques ou encore des compositions pharmaceutiques à appliquer sur la peau, les semi-muqueuses et/ou les muqueuses.

L'invention est illustrée plus en détail dans les exemples suivants.

### EXEMPLE 1

On prépare un rouge à lèvres fluide ayant la composition suivante :
- Oxyde de fer brun 4 g
- p-hydroxybenzoate de méthyle sodé 0,4 g
- Mélange de cire de polyéthylène, polytétrafluoroéthylène en émulsion dans un mélange eau/isopropanol (50/47/3) 4,5 g
- Copolymère acide méthacrylique/méthacrylate de méthoxy polyéthylène glycol/méthacrylate de lauryle en solution à 40% dans eau/PPG (50/50) (dispersant) 0,06g
- Copolymère acrylique en émulsion aqueuse vendu sous la dénomination "NEOCRYL A-1090" par la Société ZENECA 50 g
- Copolymère acide acrylique/acrylate d'éthyle en émulsion aqueuse à 28% non stabilisée (gélifiant acrylique) 5,35 g
- Solution à 60% de polydiméthylsiloxane oxyéthyléné oxypropyléné dans le dipropylèneglycol monométhyl éther (anti-mousse) 0,1 g
- Alcool éthylique à 96° non dénaturé 5 g
- 1,2-pentanediol 3 g
- Eau déminéralisée stérilisée q.s. 100 g

a) On broie tout d'abord les pigments dans un mélange contenant du 1,2-pentanediol et de l'eau.
b) On mélange la cire téflonnée dans la dispersion de polymère à température ambiante.
c) Une partie de l'eau, de l'alcool et du p-hydroxybenzoate de méthyle sodé sont mélangés entre eux, puis ajoutés à la dispersion de polymère obtenue en b).

On ajoute à ce mélange obtenu en c) l'anti-mousse puis le mélange a), sous agitation, à température ambiante, ainsi que le dispersant.

On complète avec l'eau, l'alcool et le p-hydroxybenzoate de méthyle sodé restants pour obtenir 100 g de composition.

Les tests d'efficacité antimicrobienne effectués sur cette formule par le test de contamination artificielle ou "Challenge-test" sur 6 germes à 2 jours, 7 jours et 14 jours, à température ambiante, ont montré que la protection antimicrobienne conférée par le système :
p-hydroxybenzoate de méthyle sodé (0,4%), alcool éthylique (5%) et 1,2-pentanediol (3%) était satisfaisante car l'ensemble des 6 germes inoculés *Escherichia Coli*, *Pseudomonas Aeruginosa, Staphylococcus Aureus, Enterococcus Faecalis, Candida Albicans et Aspergillus Niger,* sont décontaminés 7 jours après l'inoculation.

Les résultats sont les suivants.

| GERMES | Inoculum germes/gramme | Vieillissement à température ambiante | | |
|---|---|---|---|---|
| | | 2 J | 7 J | 14 J |
| ESCHERICHIA COLI | 5,6.10⁶ | <2,0.10² | < 2,0.10 | < 2,0.10² |
| PSEUDOMONAS AERUGINOSA | 3,8.10⁶ | <2,0.10 | <2,0.10² | <2,0.10² |
| STAPHYLOCOCCUS AUREUS | 1,6.10⁶ | <2,0.10² | <2,0.10² | <2,0.10² |
| ENTEROCOCCUS FAECALIS | 2,8.10⁶ | 6,0.10⁴ | <2,0.10² | <2,0.10² |
| CANDIDA ALBICANS | 2,4.10⁶ | <2,0.10² | <2,0.10² | <2,0.10² |
| ASPERGILLUS NIGER | 1,6.10⁶ | 2,6.10⁴ | <2,0.10² | <2,0.10² |

### EXEMPLES 2 à 4

On a également testé des formules de même composition que celle de l'exemple 1 en faisant varier les quantités des constituants du système de protection antimicrobienne comme indiqué ci-dessous.

| Formule | p-hydroxybenzoate de méthyle sodé | 1,2-pentanediol | alcool éthylique | Protection anti-microbienne |
|---|---|---|---|---|
| | | | | |
| Ex. 2 | 0,4% | 5% | - | acceptable |
| Ex. 3 | 0,4% | 5% | 5% | satisfaisant |
| Ex. 4 | 0,4% | 5% | 3% | satisfaisant |

La protection antimicrobienne a été évaluée de la même façon que dans l'exemple 1, sur les mêmes germes.

La protection antimicrobienne est acceptable si au plus 2 des 6 germes testés ne sont décontaminés qu'après 14 jours. Elle est satisfaisante si l'ensemble des 6 germes inoculés sont décontaminés 7 jours après l'inoculation.

Les résultats sont indiqués ci-après. Ils montrent que les systèmes de protection antimicrobienne des formules des exemples 2 à 4 selon l'invention assurent de façon surprenante à ces formules une protection antimicrobienne efficace malgré la faible quantité de conservateur utilisée, à savoir 0,4% de p-hydroxybenzoate de méthyle sodé.

### Exemple 2

| GERMES | Inoculum germes/gramme | Vieillissement à température ambiante | | |
|---|---|---|---|---|
| | | 2 J | 7 J | 14 J |
| ESCHERICHIA COLI | 3,3.10⁶ | <2,0.10² | <2,0. 10² | < 2,0.10² |
| PSEUDOMONAS AERUGINOSA | 2,8.10⁶ | <2,0.10² | <2,0. 10² | < 2,0. 10² |
| STAPHYLOCOCCUS AUREUS | 2,7.10⁶ | <2,0.10² | < 2,0. 10² | < 2,0. 10² |
| ENTEROCOCCUS FAECALIS | 1,2.10⁶ | 2,2.10⁵ | 8,0. 10² | < 2,0. 10² |
| CANDIDA ALBICANS | 2,4.10⁶ | <2,0.10² | <2,0. 10² | < 2,0. 10² |
| ASPERGILLUS NIGER | 1,8.10⁶ | 3,0.10⁵ | <2,0. 10² | < 2,0.10² |

### Exemple 3

| GERMES | Inoculum germes/gramme | Vieillissement à température ambiante | | |
|---|---|---|---|---|
| | | 2 J | 7 J | 14 J |
| ESCHERICHIA COLI | 2,9.10⁶ | <2,0.10² | <2,0.10² | < 2,0.10² |
| PSEUDOMONAS AERUGINOSA | 1,1.10⁶ | <2,0.10² | <2,0. 10² | < 2,0. 10² |
| STAPHYLOCOCCUS AUREUS | 1,5.10⁶ | <2,0.10² | <2,0.10² | < 2,0. 10² |
| ENTEROCOCCUS FAECALIS | 2,2.10⁶ | <2,0.10⁴ | < 2,0.10² | < 2,0.10² |
| CANDIDA ALBICANS | 3,6.10⁶ | <2,0.10² | <2,0. 10² | < 2,0. 10² |
| ASPERGILLUS NIGER | 2,8.10⁶ | 6,8.10³ | <2,0.10² | < 2,0.10² |

### Exemple 4

| GERMES | Inoculum germes/gramme | Vieillissement à température ambiante | | |
|---|---|---|---|---|
| | | 2 J | 7 J | 14 J |
| ESCHERICHIA COLI | 5,6.10⁶ | <2,0.10² | <2,0.10² | < 2,0.10² |
| PSEUDOMONAS AERUGINOSA | 3,8.10⁶ | <2,0.10² | <2,0.10² | < 2,0.10² |
| STAPHYLOCOCCUS AUREUS | 1,6.10⁶ | <2,0.10² | <2,0.10² | < 2,0.10² |
| ENTEROCOCCUS FAECALIS | 2,8.10⁶ | 7,5.10³ | <2,0.10² | < 2,0.10² |
| CANDIDA ALBICANS | 2,4.10⁶ | <2,0.10² | <2,0.10² | < 2,0.10² |
| ASPERGILLUS NIGER | 1,6.10⁶ | 1,2.10⁴ | <2,0.10² | < 2,0.10² |

### EXEMPLES COMPARATIFS 5 à 11

On a testé des formules de même composition que celle de l'exemple 1, mais en remplaçant le système de protection antimicrobienne de l'invention par des systèmes conservateurs classiques tels qu'indiqués ci-dessous.

| Formule | p-hydroxbenzoate de méthyle sodé | alcool éthylique | Germal 115 * | Digluconate de chlorhexidine | Chlorobutanol | Chlorphénésine | Hydroxyméthyl glycinate | Protection anti-microbienne |
|---|---|---|---|---|---|---|---|---|
| | | | | | | | | |
| Ex. 5 | 0,4% | 5% | - | - | - | - | - | insuffisante |
| Ex. 6 | - | | 0,3% | - | - | 0,3% | - | insuffisante |
| Ex. 7 | 0,4% | | 0,3% | - | - | - | - | insuffisante |
| Ex. 8 | 0,4% | | - | 0,25% | - | - | - | insuffisante |
| Ex. 9 | 0,4% | | - | 1% | - | - | - | insuffisante |
| Ex. 10 | 0,4% | | - | - | 0,5% | - | - | insuffisante |
| Ex. 11 | 0,4% | | - | - | - | - | 1% | insuffisante |

| | | | | | | | | |
|---|---|---|---|---|---|---|---|---|
| ∗ Germal 115 : imidazolidinyl urée. | | | | | | | | |

La protection antimicrobienne des formules a été évaluée par le test de contamination artificielle ou "Challenge-test", comme dans les exemples précédents, sur les mêmes germes.

Les résultats sont jugés insuffisants.

Ils sont indiqués ci-dessous.

### Exemple 5

| GERMES | Inoculum germes/gramme | Vieillissement à température ambiante | | |
|---|---|---|---|---|
| | | 2 J | 7 J | 14 J |
| ESCHERICHIA COLI | 3,6.10⁶ | 1,0.10⁴ | <2,0.10² | < 2,0.10² |
| PSEUDOMONAS AERUGINOSA | 2,0.10⁶ | <2,0.10² | <2,0.10² | < 2,0.10² |
| STAPHYLOCOCCUS AUREUS | 1,4.10⁶ | 1,2.10⁶ | 9,2.10³ | < 2,0.10² |
| ENTEROCOCCUS FAECALIS | 2,4.10⁶ | 2,9.10⁶ | 3,2.10⁶ | 1,0.10⁶ |
| CANDIDA ALBICANS | 1,9.10⁶ | 3,6.10³ | <2,0.10² | < 2,0.10² |
| ASPERGILLUS NIGER | 1,2.10⁶ | 1,0.10⁶ | <2,0.10² | < 2,0. 10² |

### Exemple 6

| GERMES | Inoculum germes/gramme | Vieillissement à température ambiante | | |
|---|---|---|---|---|
| | | 2 J | 7 J | 14 J |
| ESCHERICHIA COLI | 4,0.10⁶ | <2,0.10² | <2,0.10² | < 2,0.10² |
| PSEUDOMONAS AERUGINOSA | 5,2.10⁶ | <2,0.10² | <2,0. 10² | < 2,0. 10² |
| STAPHYLOCOCCUS AUREUS | 1,5.10⁶ | 3,6.10⁵ | 6,0.10² | < 2,0.10² |
| ENTEROCOCCUS FAECALIS | 2,2.10⁶ | 2,6.10⁶ | 4,2.10⁵ | 1,8.10⁴ |
| CANDIDA ALBICANS | 3,6.10⁶ | 9,4.10⁴ | 4,0.10² | < 2,0. 10² |
| ASPERGILLUS NIGER | 2,2.10⁶ | 2,0.10⁶ | 1,8.10⁶ | 4,5.10⁴ |

### Exemple 7

| GERMES | Inoculum germes/gramme | Vieillissement à température ambiante | | |
|---|---|---|---|---|
| | | 2 J | 7 J | 14 J |
| ESCHERICHIA COLI | 4,0.10⁶ | 4,6.10⁴ | < 2,0.10² | < 2,0.10² |
| PSEUDOMONAS AERUGINOSA | 5,2.10⁶ | <2,0.10² | < 2,0.10² | < 2,0.10² |
| STAPHYLOCOCCUS AUREUS | 1,5.10⁶ | 8,6.10⁵ | 3,6.10⁴ | < 2,0.10² |
| ENTEROCOCCUS FAECALIS | 2,2.10⁶ | 2,8.10⁶ | 2,8:10⁶ | 8,4.10⁵ |
| CANDIDA ALBICANS | 3,6.10⁶ | 1,4.10⁶ | <2,0.10² | < 2,0.10² |
| ASPERGILLUS NIGER | 2,2.10⁶ | 1,6.10⁶ | < 2,0.10² | < 2,0.10² |

### Exemple 8

| GERMES | Inoculum germes/gramme | Vieillissement à température ambiante | | |
|---|---|---|---|---|
| | | 2 J | 7 J | 14 J |
| ESCHERICHIA COLI | 4,0.10⁶ | 8,0.10⁵ | 1,4, 10⁴ | < 2,0.10² |
| PSEUDOMONAS AERUGINOSA | 5,2.10⁶ | 4,0.10² | < 2,0.10² | <2,0.10² |
| STAPHYLOCOCCUS AUREUS | 1,5.10⁶ | 7,0.10⁵ | 6,5.10⁵ | 1,2.10⁵ |
| ENTEROCOCCUS FAECALIS | 2,2.10⁶ | 3,1.10⁶ | 4,0.10⁶ | 3,2.10⁶ |
| CANDIDA ALBICANS | 3,6.10⁶ | 7,8.10⁴ | < 2,0.10² | <2,0.10² |
| ASPERGILLUS NIGER | 2,2.10⁶ | 8,8.10⁵ | <2,0.10² | < 2,0.10² |

### Exemple 9

| GERMES | Inoculum germes/gramme | Vieillissement à température ambiante | | |
|---|---|---|---|---|
| | | 2 J | 7 J | 14 J |
| ESCHERICHIA COLI | 2,5.10⁶ | 1,8.10⁵ | < 2,0.10² | <2,0.10² |
| PSEUDOMONAS AERUGINOSA | 1,4.10⁶ | 1,8.10⁴ | < 2,0.10² | <2,0.10² |
| STAPHYLOCOCCUS AUREUS | 2,1.10⁶ | 1,6.10⁶ | 1,3.10⁶ | 2,1.10⁴ |
| ENTEROCOCCUS FAECALIS | 4,3.10⁶ | 2,0.10⁷ | 3,3.10⁶ | 2,0.10⁶ |
| CANDIDA ALBICANS | 1,1.10⁶ | 6,0.10² | < 2,0.10² | <2,0.10² |
| ASPERGILLUS NIGER | 1,6.10⁷ | 1,6.10⁵ | < 2,0.10² | <2,0.10² |

### Exemple 10

| GERMES | Inoculum germes/gramme | Vieillissement à température ambiante | | |
|---|---|---|---|---|
| | | 2 J | 7 J | 14 J |
| ESCHERICHIA COLI | 4,2.10⁶ | 1,8.10⁶ | < 2,0.10² | < 2,0.10² |
| PSEUDOMONAS AERUGINOSA | 1,9.10⁶ | < 2,0.10² | < 2,0.10² | < 2,0.10² |
| STAPHYLOCOCCUS AUREUS | 1,6.10⁶ | 1,8.10⁶ | 1,2.10⁶ | 5,4.10⁴ |
| ENTEROCOCCUS FAECALIS | 2,0.10⁶ | 2,3.10⁶ | 2,0.10⁶ | 2,0.10⁶ |
| CANDIDA ALBICANS | 1,4.10⁶ | 8,0.10⁴ | <2,0.10² | <2,0.10² |
| ASPERGILLUS NIGER | 1,0.10⁶ | 2,6.10⁶ | 1,4.10³ | < 2,0.10² |

### Exemple 11

| GERMES | Inoculum germes/gramme | Vieillissement à température ambiante | | |
|---|---|---|---|---|
| | | 2 J | 7 J | 14 J |
| ESCHERICHIA COLI | 2,5.10⁶ | <2,0.10² | <2,0.10² | <2,0.10² |
| PSEUDOMONAS AERUGINOSA | 1,4.10⁶ | <2,0.10² | < 2,0.10² | <2,0.10² |
| STAPHYLOCOCCUS AUREUS | 2,1.10⁶ | 5,0.10⁵ | < 2,0.10² | < 2,0.10² |
| ENTEROCOCCUS FAECALIS | 4,3.10⁶ | 2,5.10⁶ | 2,5.10⁶ | 1,6.10⁵ |
| CANDIDA ALBICANS | 1,1.10⁶ | <2,0.10² | <2,0.10² | < 2,0.10² |
| ASPERGILLUS NIGER | 1,6.10⁷ | <2,0.10² | < 2,0.10² | <2,0.10² |

Les tests d'efficacité antimicrobienne effectués sur les formules contenant des systèmes conservateurs classiques ont montré que la protection antimicrobienne conférée par ces systèmes conservateurs était insuffisante, même lorsqu'on utilise le digluconate de chlorhexidine, qui est connu pour être un excellent conservateur.

### EXEMPLE 12

On prépare un rouge à lèvres fluide de composition suivante :
- Oxyde de fer brun 4 g
- 1,2-pentanediol 3 g
- Alcool éthylique 5 g
- p-hydroxybenzoate de méthyle sodé 0,4 g
- Dispersion aqueuse de polyuréthanne à 49% de matière solide, vendue sous la dénomination "SANCURE 2255" par la Société SANCOR qs 100 g

Le mode opératoire est le même que dans l'exemple 1.

### EXEMPLE 13

On prépare un maquillage semi-permanent du corps conformément à l'exemple 1, contenant éventuellement une quantité suffisante de parfum.

### EXEMPLE 14

On prépare un rouge à lèvres fluide identique à l'exemple 1, sauf que l'on remplace le copolymère acrylique "NEOCRYL A-1090" par le "NEOCRYL A-523" qui est un copolymère acrylique en émulsion aqueuse à 60% de matière solide vendu par la Société ZENECA.

## Revendications

1. Utilisation dans une composition cosmétique ou dermatologique susceptible d'être appliquée sur la peau, les fibres kératiniques, les semi-muqueuses et/ou les muqueuses, d'une dispersion aqueuse de particules de polymère filmogène associée à un système de protection antimicrobienne, notamment antibactérienne et/ou antifongique, comprenant du 1,2-pentanediol.

2. Utilisation pour maquiller, protéger et/ou traiter non thérapeutiquement et/ou pour la fabrication d'une composition destinée à traiter thérapeutiquement la peau, les fibres kératiniques, les semi-muqueuses et/ou les muqueuses, en particulier les lèvres du visage ou le corps, d'une dispersion aqueuse de particules de polymère filmogène associée à un système de protection antimicrobienne, notamment antibactérienne et/ou antifongique, comprenant du 1,2-pentanediol.

3. Utilisation selon l'une des revendications précédentes, en vue d'obtenir un film brillant, non-collant après séchage, sans transfert et/ou longue tenue.

4. Utilisation selon l'une des revendications précédentes, dans une composition de maquillage du visage ou du corps, notamment un rouge à lèvres, un fond de teint, un fard à joues ou fard à paupières, un mascara, un eye-liner, un vernis à ongles, dans une composition de soin, dans une composition solaire et/ou autobronzante, dans une composition dermatologique ou pharmaceutique, à appliquer sur la peau, les semi-muqueuses et/ou les muqueuses.

5. Utilisation selon l'une des revendications précédentes, dans une composition de rouge à lèvres ou de soin des lèvres, ou de maquillage du corps.

6. Utilisation selon l'une des revendications précédentes, dans laquelle le polymère filmogène est choisi parmi les polyuréthannes anioniques, cationiques, non ioniques ou amphotères, les polyuréthannes acryliques, les polyuréthannes-polyvinylpyrrolidones, les polyester-polyuréthannes, les polyéther-polyuréthannes, les polyurées, les polyurée-polyuréthannes, les polyesters, les polyesters amides, les polyesters à chaîne grasse, les polyamides, les résines époxyesters, les polymères et/ou copolymères acryliques et/ou vinyliques, les copolymères acryliques/ silicones, les copolymères nitrocellulose/acryliques, les polymères d'origine naturelle, éventuellement modifiés, les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes, et leurs mélanges.

7. Utilisation selon la revendication 6, dans laquelle le polymère filmogène est choisi parmi les polymères et copolymères acryliques et/ou vinyliques.

8. Utilisation selon la revendication 6, dans laquelle le polymère filmogène est un copolymère choisi parmi les copolymères acide (méth) acrylique / (méth)acrylate, acide (méth)acrylique / α-méthylstyrène, (méth)acrylate / styrène, acide (méth)acrylique / styrène, (méth)acrylate/(méth)acrylate et (méth)acrylate / α-méthylstyrène.

9. Utilisation selon la revendication 6 ou 7, dans laquelle le polymère filmogène est un copolymère issu de la copolymérisation de monomères méthacrylates d'alkyle en C₁-C₈, éventuellement associés à l'acide acrylique, au styrène et à l'α-méthylstyrène.

10. Utilisation selon l'une des revendications précédentes, dans laquelle la taille des particules de polymères en dispersion aqueuse est comprise entre 5 et 500 nm, de préférence entre 20 et 150 nm.

11. Utilisation selon l'une des revendications précédentes, dans laquelle le système de protection antimicrobienne comprend 0,1 à 10% en poids de 1,2-pentanediol, 0 à 5% en poids de p-hydroxybenzoate de méthyle sodé et/ou 0 à 40% en poids d'alcool éthylique.

12. Utilisation selon la revendication 11, dans laquelle le système de protection antimicrobienne comprend 1 à 7% en poids de 1,2-pentanediol, 0,05 à 2% en poids de p-hydroxybenzoate de méthyle sodé et/ou 0,5 à 30% en poids d'alcool éthylique.

13. Utilisation selon la revendication 12, dans laquelle le système de protection antimicrobienne comprend 3 à 5% en poids de 1,2-pentanediol, 0,2 à 1% en poids de p-hydroxybenzoate de méthyle sodé et/ou 1 à 10% en poids d'alcool éthylique.

14. Composition cosmétique ou dermatologique susceptible d'être appliquée sur la peau, les fibres kératiniques, les semi-muqueuses et/ou les muqueuses, caractérisée en ce qu'elle comprend une dispersion aqueuse de particules de polymère filmogène associée à un système de protection antimicrobienne, notamment antibactérienne et/ou antifongique, comprenant du 1,2-pentanediol.

15. Composition selon la revendication 14, se présentant sous la forme d'une composition de maquillage, d'une composition de soin, d'une composition solaire ou autobronzante, d'une composition dermatologique ou pharmaceutique, à appliquer sur la peau, les fibres kératiniques, les semi-muqueuses et/ou les muqueuses.

16. Composition selon la revendication 14 ou 15, se présentant sous la forme d'un rouge à lèvres, un fond de teint, un fard à joues ou fard à paupières, un eye-liner, un mascara, un vernis à ongles, un produit de maquillage du corps.

17. Composition de maquillage des lèvres ou du corps selon la revendication 14.

18. Composition selon l'une des revendications 14 à 17, caractérisée par le fait que le polymère filmogène est choisi parmi les polyuréthannes anioniques, cationiques, non ioniques ou amphotères, les polyuréthannes acryliques, les polyuréthannes-polyvinylpyrrolidones, les polyester-polyuréthannes, les polyéther-polyuréthannes, les polyurées, les polyurée-polyuréthannes, les polyesters, les polyesters amides, les polyesters à chaîne grasse, les polyamides, les résines époxyesters, les polymères et/ou copolymères acryliques et/ou vinyliques, les copolymères acryliques/ silicones, les copolymères nitrocellulose/acryliques, les polymères d'origine naturelle, éventuellement modifiés, les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes, et leurs mélanges.

19. Composition selon la revendication 18, caractérisée par le fait que le polymère filmogène est choisi parmi les polymères et copolymères vinyliques et/ou acryliques.

20. Composition selon la revendication 19, caractérisée par le fait que le polymère filmogène est un copolymère choisi parmi les copolymères acide (méth)acrylique / (méth)acrylate, acide (méth) acrylique / α-méthylstyrène, (méth)acrylate / styrène, acide (méth) acrylique / styrène, (méth )acrylate / (méth)acrylate et (méth)acrylate /α-méthylstyrène.

21. Composition selon la revendication 19 ou 20, caractérisée par le fait que le polymère filmogène est un copolymère issu de la copolymérisation de monomères méthacrylates d'alkyle en C₁-C₈, éventuellement associés à l'acide acrylique, au styrène et à l'α-méthylstyrène.

22. Composition selon l'une des revendications 14 à 21, caractérisée par le fait que la taille des particules de polymères en dispersion aqueuse est comprise entre 5 et 500 nm, de préférence entre 20 et 150 nm.

23. Composition selon l'une des revendications 14 à 22, caractérisée par le fait que le système de protection antimicrobienne comprend 0,1 à 10% en poids de 1,2-pentanediol, 0 à 5% en poids de p-hydroxybenzoate de méthyle sodé et/ou 0 à 40% en poids d'alcool éthylique.

24. Composition selon la revendication 23, caractérisée par le fait que le système de protection antimicrobienne comprend 1 à 7% en poids de 1,2-pentanediol, 0,05 à 2% en poids de p-hydroxybenzoate de méthyle sodé et/ou 0,5 à 30% en poids d'alcool éthylique.

25. Composition selon la revendication 24, caractérisée par le fait que le système de protection antimicrobienne comprend 3 à 5% en poids de 1,2-pentanediol, 0,2 à 1% de p-hydroxybenzoate de méthyle sodé et/ou 1 à 10% en poids d'alcool éthylique.

26. Composition selon l'une des revendications 14 à 25, caractérisée par le fait qu'elle comprend en outre au moins un colorant hydrosoluble et/ou au moins un pigment et/ou au moins une charge et/ou au moins un agent nacrant.

27. Composition selon l'une des revendications 14 à 26, caractérisée par le fait qu'elle comprend, de plus, au moins un additif choisi parmi les agents épaississants, les dispersants, les agents anti-mousses, les huiles, les cires et les actifs cosmétiques ou dermatologiques.

28. Procédé de protection antimicrobienne d'une composition contenant une dispersion aqueuse de particules de polymère filmogène, consistant à introduire dans la composition un système de protection antimicrobienne, notamment antibactérienne et/ou antifongique, comprenant du 1,2-pentanediol.

## Claims

1. Use in a cosmetic or dermatological composition which can be applied to the skin, keratin fibres, semi-mucous membranes and/or mucous membranes, of an aqueous dispersion of film-forming polymer particles combined with an antimicrobial protection system, in particular an antibacterial and/or antifungal system, comprising 1,2-pentanediol.

2. Use for making up, protecting and/or non-therapeutically treating and/or for the manufacture of a composition intended to therapeutically treat the skin, keratin fibres, semi-mucous membranes and/or mucous membranes, in particular the facial lips or the body, of an aqueous dispersion of film-forming polymer particles combined with an antimicrobial protection system, in particular an antibacterial and/or antifungal system, comprising 1,2-pentanediol.

3. Use according to either of the preceding claims, in order to obtain a glossy film which is non-sticky after drying, transfer-resistant and/or long-lasting.

4. Use according to one of the preceding claims, in a composition for making up the face or the body, in particular a lipstick, a foundation, a blusher, an eyeshadow, a mascara, an eyeliner or a nail varnish, in a care composition, in an antisun and/or self-tanning composition or in a dermatological or pharmaceutical composition to be applied to the skin, semi-mucous membranes and/or mucous membranes.

5. Use according to one of the preceding claims, in a lipstick or lip care composition or a body make-up composition.

6. Use according to one of the preceding claims, in which the film-forming polymer is chosen from anionic, cationic, nonionic or amphoteric polyurethanes, acrylic polyurethanes, polyurethane-polyvinylpyrrolidones, polyester-polyurethanes, polyether-polyurethanes, polyureas, polyurea-polyurethanes, polyesters, polyesteramides, fatty-chain polyesters, polyamides, epoxy ester resins, acrylic and/or vinyl polymers and/or copolymers, acrylic/silicone copolymers, nitrocellulose/acrylic copolymers, polymers of natural origin, which are optionally modified, polymers resulting from the radical polymerization of one or more radical monomers, inside and/or partially at the surface of pre-existing particles of at least one polymer chosen from the group consisting of polyurethanes, polyureas, polyesters, polyesteramides and/or alkyds, and mixtures thereof.

7. Use according to Claim 6, in which the film-forming polymer is chosen from acrylic and/or vinyl polymers and copolymers.

8. Use according to Claim 6, in which the film-forming polymer is a copolymer chosen from (meth)acrylic acid/(meth)acrylate, (meth)acrylic acid/α-methylstyrene, (meth)acrylate/styrene, (meth)acrylic acid/styrene, (meth)acrylate/(meth)acrylate and (meth)acrylate/α-methylstyrene copolymers.

9. Use according to Claims 6 or 7, in which the film-forming polymer is a copolymer derived from the copolymerization of C₁-C₈ alkyl methacrylate monomers, optionally combined with acrylic acid, with styrene and with α-methylstyrene.

10. Use according to one of the preceding claims, in which the size of the polymer particles in aqueous dispersion is between 5 and 500 nm, preferably between 20 and 150 nm.

11. Use according to one of the preceding claims, in which the antimicrobial protection system comprises 0.1 to 10% by weight of 1,2-pentanediol, 0 to 5% by weight of sodium methyl p-hydroxybenzoate and/or 0 to 40% by weight of ethyl alcohol.

12. Use according to Claim 11, in which the antimicrobial protection system comprises 1 to 7% by weight of 1,2-pentanediol, 0.05 to 2% by weight of sodium methyl p-hydroxybenzoate and/or 0.5 to 30% by weight of ethyl alcohol.

13. Use according to Claim 12, in which the antimicrobial protection system comprises 3 to 5% by weight of 1,2-pentanediol, 0.2 to 1% by weight of sodium methyl p-hydroxybenzoate and/or 1 to 10% by weight of ethyl alcohol.

14. Cosmetic or dermatological composition which can be applied to the skin, keratin fibres, semi-mucous membranes and/or mucous membranes, characterized in that it comprises an aqueous dispersion of film-forming polymer particles combined with an antimicrobial protection system, in particular an antibacterial and/or antifungal system, comprising 1,2-pentanediol.

15. Composition according to Claim 14, which is in the form of a make-up composition, a care composition, an antisun or self-tanning composition or a dermatological or pharmaceutical composition, to be applied to the skin, keratin fibres, semi-mucous membranes and/or mucous membranes.

16. Composition according to Claim 14 or 15, which is in the form of a lipstick, a foundation, a blusher, an eyeshadow, an eyeliner, a mascara, a nail varnish or a make-up product for the body.

17. Make-up composition for the lips or the body according to Claim 14.

18. Composition according to one of Claims 14 to 17, characterized in that the film-forming polymer is chosen from anionic, cationic, nonionic or amphoteric polyurethanes, acrylic polyurethanes, polyurethane-polyvinylpyrrolidones, polyester-polyurethanes, polyether-polyurethanes, polyureas, polyurea-polyurethanes, polyesters, polyesteramides, fatty-chain polyesters, polyamides, epoxy ester resins, acrylic and/or vinyl polymers and/or copolymers, acrylic/silicone copolymers, nitrocellulose/acrylic copolymers, polymers of natural origin, which are optionally modified, polymers resulting from the radical polymerization of one or more radical monomers, inside and/or partially at the surface of pre-existing particles of at least one polymer chosen from the group consisting of polyurethanes, polyureas, polyesters, polyesteramides and/or alkyds, and mixtures thereof.

19. Composition according to Claim 18, characterized in that the film-forming polymer is chosen from vinyl and/or acrylic polymers and copolymers.

20. Composition according to Claim 19, characterized in that the film-forming polymer is a copolymer chosen from (meth)acrylic acid/(meth)acrylate, (meth)acrylic acid/α-methylstyrene, (meth)acrylate/styrene, (meth)acrylic acid/styrene, (meth)acrylate/(meth)acrylate and (meth)acrylate/α-methylstyrene copolymers.

21. Composition according to Claim 19 or 20, characterized in that the film-forming polymer is a copolymer derived from the copolymerization of C₁-C₈ alkyl methacrylate monomers, optionally combined with acrylic acid, with styrene and with α-methylstyrene.

22. Composition according to one of Claims 14 to 21, characterized in that the size of the polymer particles in aqueous dispersion is between 5 and 500 nm, preferably between 20 and 150 nm.

23. Composition according to one of Claims 14 to 22, characterized in that the antimicrobial protection system comprises 0.1 to 10% by weight of 1,2-pentanediol, 0 to 5% by weight of sodium methyl p-hydroxybenzoate and/or 0 to 40% by weight of ethyl alcohol.

24. Composition according to Claim 23, characterized in that the antimicrobial protection system comprises 1 to 7% by weight of 1,2-pentanediol, 0.05 to 2% by weight of sodium methyl p-hydroxybenzoate and/or 0.5 to 30% by weight of ethyl alcohol.

25. Composition according to Claim 24, characterized in that the antimicrobial protection system comprises 3 to 5% by weight of 1,2-pentanediol, 0.2 to 1% by weight of sodium methyl p-hydroxybenzoate and/or 1 to 10% by weight of ethyl alcohol.

26. Composition according to one of Claims 14 to 25, characterized in that it also comprises at least one water-soluble dye and/or at least one pigment and/or at least one filler and/or at least one pearlescent agent.

27. Composition according to one of Claims 14 to 26, characterized in that it also comprises at least one additive chosen from thickeners, dispersing agents, antifoaming agents, oils, waxes and cosmetic or dermatological active agents.

28. Process for the antimicrobial protection of a composition containing an aqueous dispersion of film-forming polymer particles, this process consisting in introducing into the composition an antimicrobial protection system, in particular an antibacterial and/or antifungal system, comprising 1,2-pentanediol.

## Patentansprüche

1. Verwendung einer wäßrigen Dispersion von Partikeln eines filmbildenden Polymers in Kombination mit einem antimikrobiellen und insbesondere antibakteriellen und/oder fungiziden Schutzsystem, das 1,2-Pentandiol enthält, in einer kosmetischen oder dermatologischen Zusammensetzung, die auf die Haut, Keratinfasern, Semischleimhäute und/oder Schleimhäute aufgebracht werden kann.

2. Verwendung einer wäßrigen Dispersion von Partikeln eines filmbildenden Polymers in Kombination mit einem antimikrobiellen und insbesondere antibakteriellen und/oder fungiziden Schutzsystem, das 1,2-Pentandiol enthält, zum Schminken, zum Schutz und/oder zur nicht-therapeutischen Behandlung und/oder zur Herstellung einer Zusammensetzung, die zur therapeutischen Behandlung der Haut, der Keratinfasern, der Semischleimhäute und/oder der Schleimhäute und insbesondere der Lippen oder des Körpers vorgesehen ist.

3. Verwendung nach einem der vorhergehenden Ansprüche zur Herstellung eines glänzenden Films, der nach dem Trocknen nicht klebt, sich nicht überträgt und/oder lange haftet.

4. Verwendung nach einem der vorhergehenden Ansprüche in einer Zusammensetzung zum Schminken des Gesichts oder des Körpers insbesondere in einem Lippenstift, Make-up, Wangenrouge, Lidschatten, Mascara, Eyeliner oder Nagellack, in einer Zusammensetzung zur Pflege, zum Sonnenschutz und/oder zur Selbstbräunung, oder einer dermatologischen oder pharmazeutischen Zusammensetzung, die auf die Haut, die Semischleimhäute und/oder die Schleimhäute aufgetragen werden soll.

5. Verwendung nach einem der vorhergehenden Ansprüche in einer Zusammensetzung für Lippenstifte oder zur Pflege der Lippen oder zum Schminken des Körpers.

6. Verwendung nach einem der vorhergehenden Ansprüche, wobei das filmbildende Polymer unter den anionischen, kationischen, nichtionischen oder amphoteren Polyurethanen, Acrylpolyurethanen, Polyurethanpolyvinylpyrrolidonen, Polyesterpolyurethanen, Polyetherpolyurethanen, Polyharnstoffen, Polyharnstoffpolyurethanen, Polyestern, Polyesteramiden, Polyestern mit Fettkette, Polyamiden, Epoxyesterharzen, Acryl- und/oder Vinylpolymeren, Acryl- und/oder Vinyl-Copolymeren, Acryl/Silicon-Copolymeren, Nitrocellulose/Acryl-Copolymeren, gegebenenfalls modifilzierten Polymeren natürlicher Herkunft und Polymeren, die bei der radikalischen Polymerisation eines oder mehrerer radikalisch polymerisierbarer Monomere im Inneren und/oder zum Teil an der Oberfläche von bereits vorhandenen Partikel mindestens eines Polymers erhalten werden, das unter den Polyurethanen, Polyharnstoffen, Polyestern, Polyesteramiden und/oder Alkydharzen ausgewählt ist, und deren Gemischen ausgewählt ist.

7. Verwendung nach Anspruch 6, wobei das filmbildende Polymer unter den Acryl- und/oder Vinylpolymeren und Acryl- und/oder Vinyl-Copolymeren ausgewählt ist.

8. Verwendung nach Anspruch 6, wobei das filmbildende Polymer ein Copolymer ist, das unter den Copolymeren (Meth)acrylsäure/(Meth)acrylat, (Meth)acrylsäure/α-Methylstyrol, (Meth)acrylat/Styrol, (Meth)acrylsäure/Styrol, (Meth)acrylat/(Meth)acrylat und (Meth)acrylat/α-Methylstyrol ausgewählt ist.

9. Verwendung nach Anspruch 6 oder 7, wobei das filmbildende Polymer ein Copolymer ist, das durch Copolymerisation von C₁₋₈-Alkylmethacrylatmonomeren gegebenenfalls in Kombination mit Acrylsäure, Styrol und α-Methylstyrol hergestellt ist.

10. Verwendung nach einem der vorhergehenden Ansprüche, wobei die Größe der Polymerpartikel in wäßriger Dispersion im Bereich von 5 bis 500 nm und vorzugsweise 20 bis 150 nm liegt.

11. Verwendung nach einem der vorhergehenden Ansprüche, wobei das antimikrobielle Schutzsystem 0,1 bis 10 Gew.-% 1,2-Pentandiol, 0 bis 5 Gew.-% Methyl-p-hydroxybenzoat-Natriumsalz und/oder 0 bis 40 Gew.-% Ethanol enthält.

12. Verwendung nach Anspruch 11, wobei das antimikrobielle Schutzsystem 1 bis 7 Gew.-% 1,2-Pentandiol, 0,05 bis 2 Gew.-% Methyl-p-hydroxybenzoat-Natriumsalz und/oder 0,5 bis 30 Gew.-% Ethanol enthält.

13. Verwendung nach Anspruch 12, wobei das antimikrobielle Schutzsystem 3 bis 5 Gew.-% 1,2-Pentandiol, 0,2 bis 1 Gew.-% Methyl-p-hydroxybenzoat-Natriumsalz und/oder 1 bis 10 Gew.-% Ethanol enthält.

14. Kosmetische oder dermatologische Zusammensetzung, die auf die Haut, die Keratinfasern, die Semischleimhäute und/oder die Schleimhäute aufgebracht werden kann, dadurch gekennzeichnet, daß sie eine wäßrige Dispersion von Partikeln eines filmbildenden Polymers in Kombination mit einem antimikrobiellen und insbesondere antibakteriellen und/oder fungiziden Schutzsystem enthält, das 1,2-Pentandiol enthält.

15. Zusammensetzung nach Anspruch 14, die in Form einer Zusammensetzung zum Schminken, einer Zusammensetzung zur Pflege, einer Zusammensetzung zum Sonnenschutz oder zum Selbstbräunen oder einer dermatologischen oder pharmazeutischen Zusammensetzung vorliegt, die auf die Haut, die Keratinfasern, die Semischleimhäute und/oder die Schleimhäute aufgetragen werden soll.

16. Zusammensetzung nach Anspruch 14 oder 15, die in Form von Lippenstift, Make-up, Wangenrouge, Lidschatten, Eyeliner, Mascara, Lippenstift oder als Produkt zum Schminken des Körpers vorliegt.

17. Zusammensetzung zum Schminken der Lippen oder des Körpers nach Anspruch 14.

18. Zusammensetzung nach einem der Ansprüche 14 bis 17, dadurch gekennzeichnet, daß das filmbildende Polymer unter den anionischen, kationischen, nichtionischen oder amphoteren Polyurethanen, Acrylpolyurethanen, Polyurethanpolyvinylpyrrolidonen, Polyesterpolyurethanen, Polyetherpolyurethanen, Polyharnstoffen, Polyharnstoffpolyurethanen, Polyestern, Polyesteramiden, Polyestern mit Fettkette, Polyamiden, Epoxyesterharzen, Acryl- und/oder Vinylpolymeren, Acryl- und/oder Vinyl-Copolymeren, Acryl/Silicon-Copolymeren, Nitrocellulose/Acryl-Copolymeren, gegebenenfalls modifizierten Polymeren natürlicher Herkunft und Polymeren, die bei der radikalischen Polymerisation eines oder mehrerer radikalisch polymerisierbarer Monomere im Inneren und/oder zum Teil an der Oberfläche von bereits vorhandenen Partikeln mindestens eines Polymers erhalten werden, das unter den Polyurethanen, Polyharnstoffen, Polyestern, Polyesteramiden und/oder Alkydharzen ausgewählt ist, und deren Gemischen ausgewählt ist.

19. Zusammensetzung nach Anspruch 18, dadurch gekennzeichnet, daß das filmbildende Polymer unter den Vinyl- und/oder Acryl-Polymeren und Vinyl-und/oder Acryl-Copolymeren ausgewählt ist.

20. Zusammensetzung nach Anspruch 19, dadurch gekennzeichnet, daß das filmbildende Polymer ein Copolymer ist, das unter den Copolymeren (Meth)acrylsäure/(Meth)acrylat, (Meth)acrylsäure/α-Methylstyrol, (Meth)acrylat(Styrol, (Meth)acrylsäure/Styrol, (Meth)acrylat((Meth)acrylat und (Meth)acrylat/α-Methylstyrol ausgewählt ist.

21. Zusammensetzung nach Anspruch 19 oder 20, dadurch gekennzeichnet, daß das filmbildende Polymer ein Copolymer ist, das durch Copolymerisation von C₁₋₈-Alkylmethacrylatmonomeren gegebenenfalls in Kombination mit Acrylsäure, Styrol und α-Methylstyrol hergestellt werden kann.

22. Zusammensetzung nach einem der Ansprüche 14 bis 21, dadurch gekennzeichnet, daß die Größe der Polymerpartikel in wäßriger Dispersion im Bereich von 5 bis 500 nm und vorzugsweise 20 bis 150 nm liegt.

23. Zusammensetzung nach einem der Ansprüche 14 bis 22, dadurch gekennzeichnet, daß das antimikrobielle Schutzsystem 0,1 bis 10 Gew.-% 1,2-Pentandiol, 0 bis 5 Gew.-% Methyl-p-hydroxybenzoat-Natriumsalz und/oder 0 bis 40 Gew.-% Ethanol enthält.

24. Zusammensetzung nach Anspruch 23, dadurch gekennzeichnet, daß das antimikrobielle Schutzsystem 1 bis 7 Gew.-% 1,2-Pentandiol, 0,05 bis 2 Gew.-% Methyl-p-hydroxybenzoat-Natriumsalz und/oder 0,5 bis 30 Gew.-% Ethanol enthält.

25. Zusammensetzung nach Anspruch 24, dadurch gekennzeichnet, daß das antimikrobielle Schutzsystem 3 bis 5 Gew.-% 1,2-Pentandiol, 0,2 bis 1 % Methyl-p-hydroxybenzoat-Natriumsalz und/oder 1 bis 10 Gew.-% Ethanol enthält.

26. Zusammensetzung nach einem der Ansprüche 14 bis 25, dadurch gekennzeichnet, daß sie ferner mindestens einen wasserlöslichen Farbstoff und/oder mindestens ein Pigment und/oder mindestens einen Füllstoff und/oder mindestens ein Perlglanzpigment enthält.

27. Zusammensetzung nach einem der Ansprüche 14 bis 26, dadurch gekennzeichnet, daß sie ferner einen Zusatzstoff enthält, der unter den Verdickungsmitteln, Dispergiermitteln, Antischaummitteln, Ölen, Wachsen und kosmetischen oder dermatologischen Wirkstoffen ausgewählt ist.

28. Verfahren zum antimikrobiellen Schutz einer Zusammensetzung, die eine wäßrige Dispersion von Partikeln eines filmbildenden Polymers enthält, das darin besteht, in die Zusammensetzung ein antimikrobielles und insbesondere antibakterielles und/oder fungizides Schutzsystem einzuarbeiten, das 1,2-Pentandiol enthält.
